# EUROPEAN PATENT SPECIFICATION

(11) **EP 2 281 583 B1**
(45) Date of publication and mention of the grant of the patent: **08.07.2015**
(21) Application number: 10181065.3
(22) Date of filing: 09.12.2003
(51) Int. Cl.: A61L 12/08, A45C 11/00, A45C 11/04, B65B 25/00

(54) **Contact lens packages containing additives**
Kontaktlinsenverpackungen enhaltend Additive
Emballages de lentilles de contact contenant des additifs

(30) Priority: 23.12.2002 US 436109 P
(43) Date of publication of application: 09.02.2011
(62) Divisional of application: 06076076.6
(73) Proprietor: Johnson and Johnson Vision Care, Inc., Jacksonville, FL 32256 (US)
(72) Inventor: Peck, James, Maple Grove, MN 55369 (US); Dubey, Dharmesh, Jacksonville, FL 32256 (US); Tokarski, Michael, Ponte Vedra Beach, FL 32082 (US); Zhang, Qiang, Annandale, NJ 08801 (US); Li, Yufu, Bridgewater, NJ 08807 (US); Arnold, Steven, Sparta, NJ 07871 (US)
(74) Representative: Tunstall, Christopher Stephen

(56) References cited:
- EP-A- 1 033 326
- GB-A- 2 078 760
- US-A- 4 100 309
- US-A1- 2002 046 958
- US-A1- 2002 107 324
- DATABASE WPI Section Ch, Week 197737 Thomson Scientific, London, GB; Class A96, AN 1977-65891Y XP002287714, -& JP 52 093398 A (TOA IYO DENSHI KK) 5 August 1977 (1977-08-05)

## Description

### FIELD OF THE INVENTION

This invention related to packages for storing contact lenses as well as methods of using and preparing these packages.

### BACKGROUND

Contact lenses have been used commercially to improve vision since the 1950s. At first contact lenses were made of hard materials, which were relatively easy to handle and package for use, but were uncomfortable for many patients. Later developments, gave rise to softer more comfortable lenses made of hydrophobic hydrogels, particularly silicone hydrogels. These lenses are very pliable, but due to this texture and their chemical composition, they present a number of problems with packaging.

Most contact lenses are packaged in individual blister packages having a bowl portion and a foil top, where the bowl portion is made from a hydrophobic material such as polypropylene. See U.S. Patent Nos. 4,691,820; 5,054,610; 5,337,888; 5,375,698; 5,409,104; 5,467,868; 5,515,964; 5,609,246; 5,695,049; 5,697,495; 5,704,468; 5,711,416; 5,722,536; 5,573,108; 5,823,327; 5,704,468; 5,983,608; 6,029,808; 6,044,966; and 6,401,915 for examples of such packaging. While polypropylene is resilient enough to withstand the sterilization steps of contact lens manufacture, this material has an affinity for contact lenses made of silicone hydrogels. When silicone hydrogels are packaged in polypropylene bowls, the lenses stick to the bowl and cannot be removed from the package without damaging the pliable lenses. Therefore is a need to prepare a contact lens package that has resilient properties, but does not stick to the final product. It is this need that is met by the following invention.

US 2002/046958 discloses a container for storing a silicone hydrogel contact lens in a liquid, having a bowl portion with a radius such that the radius of the lens is from about 85 percent to about 100 percent of the inner surface radius. The bowl portion has an outer section which is defined by a radius larger than the inner surface radius. Furthermore, the bowl portion has a roughness sufficient to maintain capillary attraction of the lens to the bowl portion but preventing adhesion of any portion of the front surface of the lens to the bowl portion.

### BRIEF DESCRIPTION OF THE DRAWINGS

Figure 1 illustrates the data for Lens A in different packages
Figure 2 illustrates the data for Lens B in different packages
Figure 3 illustrates the data for Lens C in different packages

### DETAILED DESCRIPTION OF THE INVENTION

This invention includes a package for storing contact lenses in a solution comprising, consisting essentially of, or consisting of, a molded base wherein the molded base comprises an additive, wherein the additive is polyvinylpyrrolidinone (PVP), wherein the package contains a contact lens, wherein the contact lens is in a solution and wherein the molded base comprises polypropylene mixed with said additive, provided that the contact lens is not a contact lens consisting of acqualfilcon A coated with polyHema.

The contact lenses include but are not limited to soft contact lenses. These devices can provide optical correction or may be cosmetic. The preferred contact lenses of the invention are soft contact lenses made from silicone elastomers or hydrogels, which include but are not limited to silicone hydrogels, and fluorohydrogels. Soft contact lens formulations are disclosed inUS 2003/0125498 A1, entitled Biomedical Devices Containing Internal wetting Agents,", US Patent No. 5,710,302, WO 9421698, EP 406161, JP 2000016905, U.S. Pat. No. 5,998,498, U.S. Patent No. 6,087,415, U.S. Pat. No. 5,760,100, U.S. Pat. No.5,776,999, U.S. Pat. No. 5,789,461, U.S. Pat. No. 5,849,811, and U.S. Pat. No. 5,965,631. The particularly preferred contact lenses are soft contact lenses made from etafilcon A, genfilcon A, lenefilcon A, polymacon, balafilcon A, lotrafilcon A. and silicone hydrogels as prepared in U.S. Pat. No. 5,998,498, U.S. Patent No. 6,087,415, U.S. Pat. No. 5,760,100, U.S. Pat. No.5,776,999, U.S. Pat. No. 5,789,461, U.S. Pat. No. 5,849,811, and U.S. Pat. No. 5,965,631. The more particularly preferred contact lenses of the invention are soft contact lenses, balafilcon A, lotrafilcon A, galyfilcon A, senofilcon A, or those made as described in US 2003/0125498 A1. The most particularly preferred contact lenses are soft contact lenses made from either galyfilcon A or senofilcon A.

The term "molded base" refers to any polymer, rubber, or plastic that can be formed into a receptacle for medical devices, where the size and shape of the base are determined by the device and other considerations known those who are skilled in the art of making or designing molded bases. For example molded bases may be individual blister packages, secondary packages, or hydrating trays. The molded base may be prepared from any number of materials provided that those materials are compatible with the chemical and physical properties of the device. Examples of suitable materials include but are not limited to polypropylene, polyethylene, nylons, olefin co- polymers, acrylics, rubbers, urethanes, polycarbonates, or fluorocarbons. The preferred materials are metallocenes polymers and co-polymers made of polypropylene, polyethylene, having a melt flow range of about 15 g/10 minutes to about 44 g/10 minutes as determined by ASTM D-1238. With respect to the shape of the molded base, examples of suitably shaped bases are disclosed in the following patents, U.S. Patent Nos. D 458,023; 4,691,820; 5,054,610; 5,337,888; 5,375,698; 5,409,104; 5,467,868; 5,515,964; 5,609,246; 5,695,049; 5,697,495; 5,704,468; 5,711,416; 5,722,536; 5,573,108; 5,823,327; 5,704,468; 5,983,608; 6,029,808; 6,044,966; and 6,401,915. As in the cited references, the molded based is sealed about the cavity that encloses the contact lens. Flexible cover sheets can be made from can be an adhesive laminate of an aluminum foil and a polypropylene film or any other extruded or co-extruded film that can be sealed to the top surface of the flange in order to form a hermetic seal for the medical device and the solution. Further, the base can be formed by any of a number of known methods which include but are not limited to injection molding, transfer molding, skin packaging, blow molding, coinjection molding, film extrusion, or film coextrusion.

As used herein the term "additive" refers to a substance that is added to the polymer, rubber, or plastic prior to forming the molded base, where the material inhibits sticking, adherence, or adhesion of the contact lenses to the molded base. The additive is mixed with the remainder of the molded base material and amount of additive present by weight percentage based on the total weight of the molded base material is polyvinylpyrolidone (1% to 5%). Polyvinylpyrolidinone has a variety of molecular weight ranges (as indicated by the KD#) and consistencies (flake, powdered/micronized). When PVP KD90 is used as an additive, it is preferred that it is powered/micronized.

The term "solution" refers to any liquid medium in which a medical device is stored. The preferred solutions are aqueous solutions contain physiological buffers. The particularly preferred solution is saline solution.

For example, it is preferred that the molded base is transparent to the degree necessary to permit visual inspection, UV sterilization or both. The preferred additive is PVP KD90 present at about 1-5 weight percent.

Further, the invention includes a method of reducing the adherence of a contact lens to its packaging, comprising, consisting essentially of, or consisting of, storing said contact lens in a solution in a package comprising, consisting essentially of, or consisting of, a molded base wherein said melded base comprises an additive, wherein said additive is polyvinylpyrrolidinone (PVP) and wherein the molded base comprises polypropylene mixed with said additive, wherein the additive is present at a concentration of 1 weight percent to 5 weight percent, provided that the contact lens is not a contact lens consisting of acqualfilcon A coated with polyHema. The terms molded base, contact lens, solution and additive all have their aforementioned meanings and preferred ranges.

When soft contact lenses are prepared, the lenses cured to a hard disc and subsequently hydrated with water to give the non-sterilized final product. During this hydration step, soft contact lenses often stick to the surface of the hydration chamber and it would useful to find a method of hydrating soft contact lenses which alleviates this problem.

Other have tried to address the problem of a medical device adhering to its packaging. For example US 2002/0046958 A1, entitled "Textured Contact Lens Package," and US 2004/0004008 A1, entitled "Contact Lens Packages," disclose solutions to this problem. Even though those methods address this problem, it is contemplated by the inventors of this patent application that the additives of this invention may be incorporated into the packaging of each of the cited references.

In order to illustrate the invention the following examples are included. These examples do not limit the invention. They are meant only to suggest a method of practicing the invention. Those knowledgeable in contact lenses as well as other specialties may find other methods of practicing the invention. However, those methods are deemed to be within the scope of this invention.

### EXAMPLES

The following abbreviations are used below
- Ampacet 40604: fatty acid amide
- ATOFINA 3924CWZ: Finacene Nucleated polypropylene having a melt flow of 55g/10 minutes, ASTM D1238. This material contains an antistat and a lubricant
- Atmer 163: fatty alkyl diethanolamine Reg. No.107043-84-5
- Dow Siloxane MB50-321: a silicone dispersion
- Epolene E43-Wax,: maleic anhydride produced by Eastman Chemical
- Erucamide: fatty acid amide Registry No. 112-84-5
- Exxon 1605: Exxon Achieve, PP1605, a metallocene polypropylene having a melt flow of 32 g/10 minutes, ASTM D-1238 (L)
- Exxon 1654: Exxon Achieve, PP1654, a metallocene isotactic polypropylene having a melt flow of 16 g/10 minutes, ASTM D-1238 (L)
- Fina EOD-001: Finacene, a metallocene and isotactic polypropylene having a melt flow of 16g/10 minutes, ASTM D1238
- Flura: Registry No.7681-49-4
- Kemamide: fatty acid amide
- Licowax: fatty acid amide
- Mica: Registry No. 12001-26-2
- Nurcrel 535 & 932: ethylene-methacrylic acid co-polymer resin Registry No. 25053-53-6
- Oleamide: fatty acid amide Registry No. 301-02-0
- polyHema: poly hydroxy ethylmethacylate having a molecular weight of greater than 1 MM Dalton
- mPDMS: 800-1000 MW monomethacryloxypropyl terminated polydimethylsiloxane
- Pluronic: polyoxypropylene-polyoxyethylene block co-polymer Registry No.106392-12-5
- PVP: poly vinyl pyrrolidinone, wherein KD# refers to different known molecular weight distributions of poly vinyl pyrrolidinone
- Simma 2: 3-methacryloxy-2-hydroxypropyloxy)propylbis (trimethylsiloxy)methylsilane
- Super-Floss anti block: slip/anti blocking agent, Registry No. 61790-53-2
- Tetronic: alkyoxylated amine 110617-70-4
- Zeospheres anti-block: slip/anti blocking agent

### Lens Preparation

- Lens A: Acquafilcon A lenses coated with polyhema having a molecular weight of about 1,000,000. See US 2002/0107324 A1, entitled "Soft Contact Lenses,", Example 27. The coating method is disclosed in US 2003/0052424 A1, entitled "Method for Correcting Articles by Mold Transfer,".
- Lens B: Contact lenses prepared as described in US 2003/0125498 A1, entitled Biomedical Devices Containing Internal wetting Agents, containing by weight percent 30% Simma 2, 19% mPDMS, 31% DMA, 6% PVP (MW 360,000), 0.8%EDGMA, 0.23% CGl81, 1.5% Norbloc, 11% PVP (MW 2,500), 0.02% Blue Hema, 0-2 ac PDMS, 29% t-amyl alcohol.
- Lens C: Contact lenses prepared as described in US 2003/0125498 A1, entitled Biomedical Devices Containing Internal wetting Agents,", containing by weight percent 28% Simma 2, 31% mPDMS, 23.5% DMA, 7% PVP (MW 360,000), 1.5%TEDGMA, 0.98% CGI 1850, 2.0% Norbloc, 6 HEMA, 0.02% Blue Hema.

### Reference Example 1

### Preparation of Packages with Different Additives

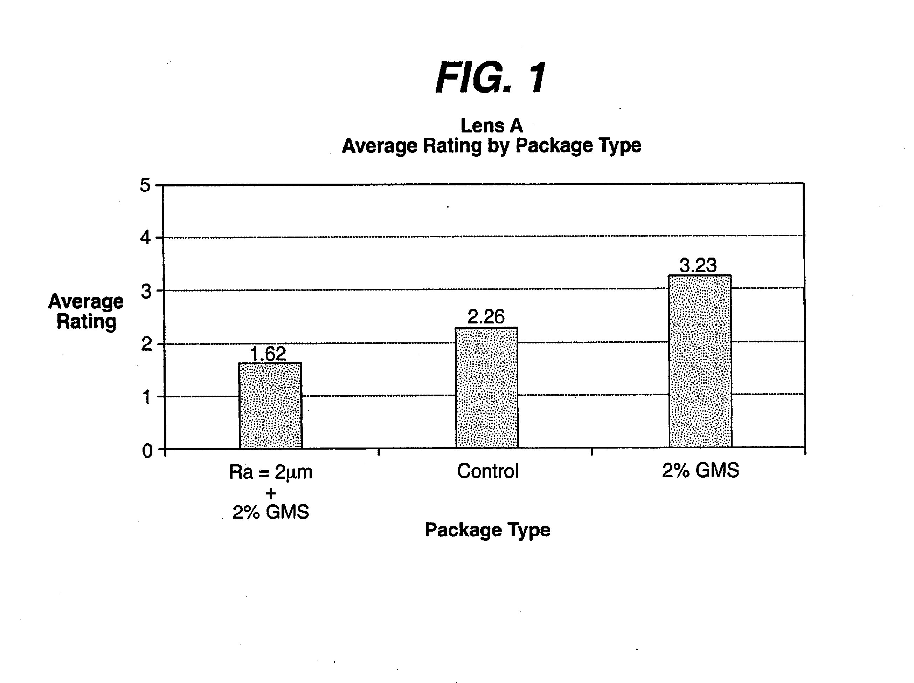

Additives (identity and amounts listed in Table 1) were mixed with polypropylene (listed below). The material was injection molded to form the base portion of a contact lens package. The configuration of the package is as illustrated in Figure1 of U.S. Pat No. 5,467,868.

Contact lenses made from acquafilcon A coated with polyhema, a silicone hydrogel, were added to individual polypropylene blister packs having different additives containing 950µL of saline solution and then the blister pack was heat sealed with an flexible cover. Lenses were visually evaluated for adhesion to the package after sterilization. The flexible cover sheet was removed and the molded base is rotated or jiggled without spilling the saline solution while a contact lens is observed to determine if it is adhered to the inner surface of the molded base. Lenses that do not adhere are free floating and pass the test. If the lenses adhere to the molded base in any manner they fail the test. The additive, its weight percentage, the number of lenses that stuck to the package, and number of lenses that were free floating are displayed in Table 1. This example illustrates that glycerol monostearate is a superior additive.

**TABLE 1**

| Polypropylene | Additive | # tested | # stuck |
|---|---|---|---|
| Exxon 1605 | none | 12 | 12 |
| Exxon 1605 | calcium stearate | 36 | 36 |
| Exxon 1605 | 2% glycerol monostearate | 36 | 3 |
| Exxon 1654 | 2% glycerol monostearate | 84 | 2 |
| Exxon 1654 | none | 12 | 12 |
| Exxon Exxeler P1020 | none | 12 | 12 |
| Fina EOD-0011 | none | 12 | 12 |
| Fina EOD-0011 | 1 % zinc stearate | 12 | 12 |
| Fina EOD-0011 | 3% zinc stearate | 12 | 12 |
| FINA 3924CW@ | antistat | 36 | 36 |

### Reference Example 2

### Consumer Test

Packages containing 2% weight percent GMS and Exxon 1605 were prepared using the method of Example 1. Contact lenses of types A, B, and C were added to individual blister packages along with 950 µL of saline solution. The filed packages were heat sealed with flexible covers and sterilized. The packaged lenses were submitted to consumers. The consumers opened the packages and evaluated the lenses for ease of removal of the lens from the package using the following criteria and grading system
1-very easy removal-Lens comes out without any problems
2-easy removal-a couple of attempts to remove the lenses, but overall there were no real problems in removal
3-moderate removal-several tries before lens comes out, neither pleased or displeased
4-difficult removal-many tries to remove with finger or nail-removal is frustrating
5-very difficult removal-many tries to remove with a finger or nail, lens damage upon removal- very unacceptable

Figure 1 illustrates the testing results for a comparison of Lens A in a polypropylene package (control), Lens A in a package containing 2.0% GMS where the package has an average surface roughness (Ra) of about 2.0 µm, and Lens A in a package containing 2.0% GMS. This figure shows that the roughened package containing GMS has the highest consumer rating.

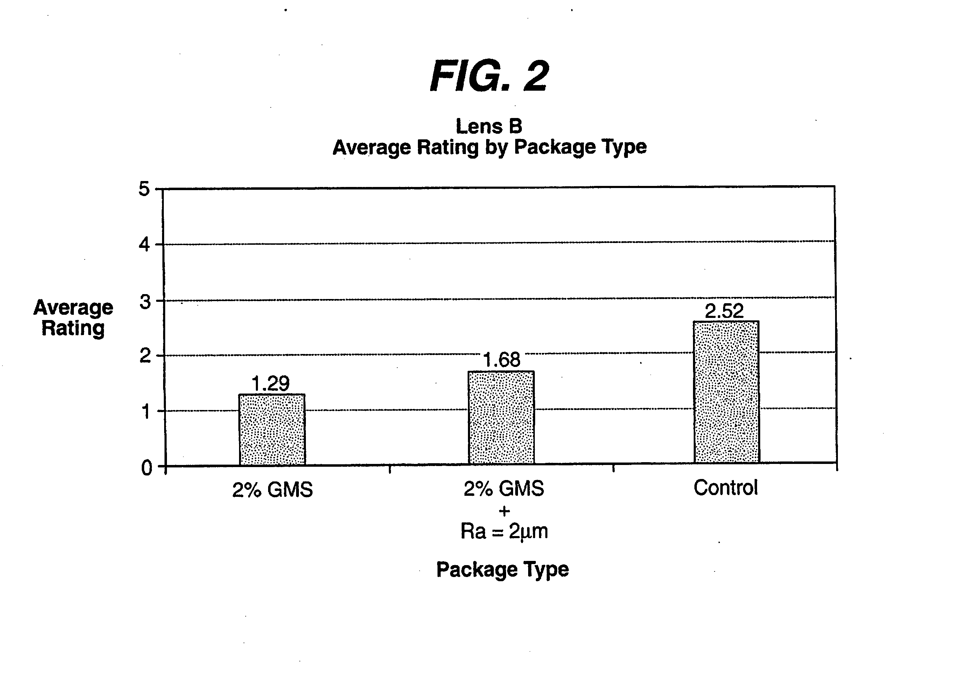

Figure 2 illustrates the testing results for a comparison of Lens B in a polypropylene package (control), Lens B in a package containing 2.0% GMS where the package has an average surface roughness (Ra) of about 2.0 µm, and Lens B in a package containing 2.0% GMS. This figure shows that the package containing 2.0 %GMS has the highest consumer rating.

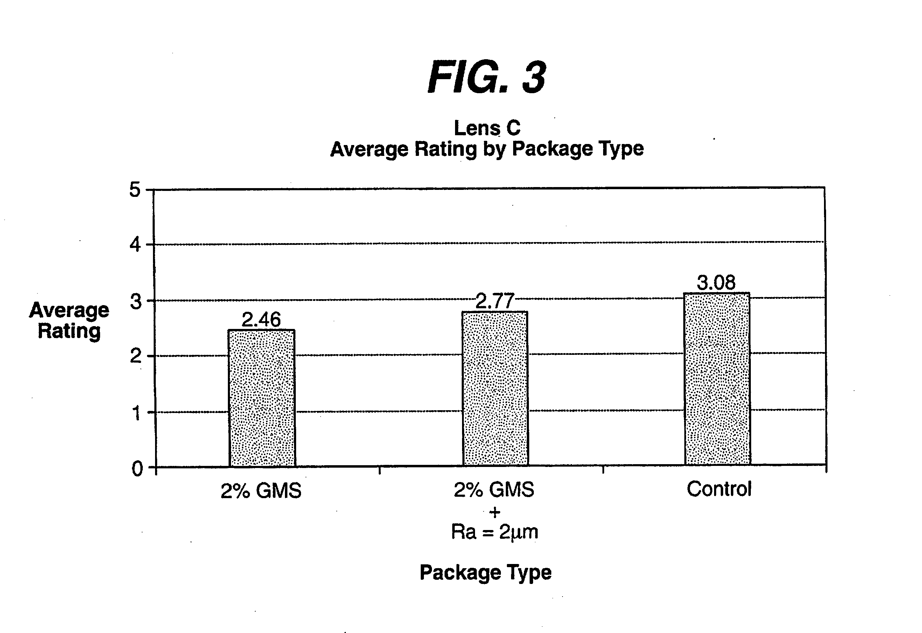

Figure 3 illustrates the testing results for a comparison of Lens C in a polypropylene package (control), Lens C in a package containing 2.0% GMS where the package has an average surface roughness (Ra) of about 2.0 µm, and Lens C in a package containing 2.% GMS. This figure shows that the package containing 2.0 %GMS has the highest consumer rating.

### Example 3

### Preparation of Packages With Different Additives

The testing methods and preparations of Reference Example 1 were repeated with different additives and lens types as per Table 2. If "(UP)" appears in an entry, that bowl of the blister is shaped as in U.S. Pat. No. D 458,023. When the term "Rough Bowl" appears, the inside surface of the bowl is roughened to an Ra of 0.5mm to 0.8mm.

**Table 2**

| Base Resin | Lens Type | Tested | Stuck | Additive |
|---|---|---|---|---|
| Exxon 1605 PP | Lens B | 15 | 13 | Calcium stearate (2%) |
| Exxon 1605 PP | Lens B | 120 | 0 | GMS (2%) |
| Exxon 1605 PP | Lens C | 30 | 0 | GMS (2%) |
| Exxon 1605 PP | Lens B | 15 | 12 | Dow Siloxane MB50-321 (10%) |
| Exxon 1605 PP | Lens B | 15 | 13 | Dow Siloxane MB50-321 (5%) |
| Exxon 1605 PP | Lens B | 57 | 50 | Ampacet 40604 99.5/.5 Erucamide |
| Ampacet 40604 PP | Lens B | 15 | 15 | Erucamide (5%) |
| Exxon 1605 PP | Lens B | 15 | 15 | Kemamide (Erucamide) (5%) |
| Exxon 1605 PP | Lens B | 15 | 12 | Superfloss anti bock (2%) |
| Exxon 1605 PP | Lens B | 15 | 15 | Zeospheres antj.block (2%) |
| Exxon 1605 PP | Lens B | 15 | 14 | Superfloss anti bock (2%) Oleamide (.2%) |
| Exxon 1605 PP | Lens B | 14 | 13 | Superfloss antj.bock (.2%) Oleamide (.2%) |
| Exxon 1605 PP | Lens B | 15 | 15 | Talc (5%) |
| Exxon 1605 PP | Lens B | 15 | 13 | Calcium carbonate (5%) |
| Exxon 1605 PP | Lens B | 15 | 14 | Zinc stearate (5% hand blend) |
| Exxon 1605 PP | Lens B | 15 | 15 | Zinc stearate (5% machine blend) |
| Exxon 1605 PP | Lens B | 15 | 14 | ATP (Vitamin E) (5%) |
| Exxon 1605 PP | Lens B | 15 | 13 | Licowax (1%) |
| Exxon 1605 PP | Lens B | 15 | 14 | Polyethyleneglycol monolaurate (5%) |
| Exxon 1605 PP | Lens B | 15 | 15 | Mica (5%) |
| Exxon 1605 PP | Lens B | 175 | 8 | Succinic Acid (5%) |
| Exxon 1605 PP | Lens B | 15 | 13 | Succinic Anhydride (5%) |
| Exxon 1605 PP | Lens B | 118 | 22 | Epolene E-43 (20% machine blend) |
| Exxon 1605 PP | Lens B | 100 | 92 | Epolene E-43 (20% machine blend) |
| Exxon 1605 PP | Lens B | 127 | 52 | Epolene E-43 (10% hand blend) |
| Exxon 1605 PP | Lens B | 130 | 16 | Epolene E-43 (10% machine blend) |
| Exxon 1605 PP | Lens C | 15 | 6 | Epolene E-43 (10% machine blend) |
| Exxon 1605 PP | Lens B | 30 | 22 | Epolene E43 (5% machine blend) |
| Exxon 1605 PP | Lens C | 15 | 3 | Epolene E-43 (5% machine blend) |
| Exxon 1605 PP | Lens B | 15 | 15 | Atmer 163 (1%) |
| Exxon 1605 PP | Lens B | 15 | 10 | MC (5%) |
| Exxon 1605 PP | Lens B | 30 | 2 | Boric Acid (5% hand blend) |
| Exxon 1605 PP | Lens B | 215 | 3 | Boric Acid (5% machine blend) |
| Exxon 1605 PP | Lens C | 15 | 0 | Boric Acid (5% machine blend) |
| Exxon 1605 PP | Lens B | 15 | 13 | Boric Acid (3% hand blend) |
| Exxon 1605 PP | Lens B | 15 | 15 | Boric Acid (2% hand blend) |
| Exxon 1605 PP | Lens B | 150 | 4 | Epolene E43 (10% machine blend) |
| Exxon 1605 PP | Lens B | 50 | 9 | Epolene E-43 (10% machine blend) |
| Exxon 1605 PP | Lens B | 50 | 15 | Epolene E-43 (10% machine blend) |
| Exxon 1605 PP | Lens B | 50 | 35 | Epolene E-43 (10% machine blend) |
| Exxon 1605 PP | Lens B | 255 | 6 | PVP K90 (5.0%) |
| Exxon 1605 PP | Lens B | 98 | 31 | PVP K90 (2.5%) |
| Exxon 1605 PP | Lens B | 98 | 49 | PVP K90 (1.25%) |
| Exxon 1605 PP | Lens B | 20 | 6 | PVP K90 (1.0%) |
| Exxon 1605 PP | Lens B | 20 | 10 | PVP K90 (.75%) |
| Exxon 1605 PP | Lens B | 20 | 17 | PVP K90 (.5%) |
| Exxon 1605 PP | Lens C | 248 | 5 | PVP K90 (5.0%) |
| Exxon 1605 PP | Lens C | 39 | 0 | PVP K90 (10%) Blended down to 5% |
| Exxon 1605 PP | Lens C | 135 | 42 | PVP K90 (2.5%) |
| Exxon 1605 PP | Lens C | 135 | 54 | PVP K90 (1.25%) |
| Exxon 1605 PP | Lens C | 70 | 42 | PVP K90 (1.0%) |
| Exxon 1605 PP | Lens C | 70 | 50 | PVP K90 (.75%) |
| Exxon 1605 PP | Lens C | 70 | 60 | PVP K90 (.5%) |
| Exxon 1605 PP | Lens B | 15 | 14 | Nucrel 535 - 10.5% acid comonomer (2%) |
| Exxon 1605 PP | Lens B | 15 | 15 | Nucrel 925 - 15% acid comonomer (3%) |
| Exxon 1605 PP | Lens C | 15 | 14 | Nucrel 535 - 10.5% acid comonomer (2%) |
| Exxon 1605 PP | Lens C | 15 | 14 | Nucrel 925 - 15% acid comonomer (3%) |
| Exxon 1605 PP | Lens B | 15 | 15 | 2% XNAP with Pluronic |
| Exxon 1605 PP | Lens C | 15 | 14 | 2% XNAP with Pluronic |
| Exxon 1605 PP | Lens B | 15 | 15 | Pluronic 1% |
| Exxon 1605 PP | Lens C | 15 | 15 | Pluronic 1% |
| Exxon 1605 PP | Lens B | 15 | 11 | 1% Tetronic |
| Exxon 1605 PP | Lens C | 15 | 15 | 1 % Tetronic |
| Exxon 1605 PP | Lens B | 15 | 15 | 1% Flura |
| Exxon 1605 PP | Lens C | 15 | 15 | 1% Flura |
| Exxon 1605 PP | Lens B | 30 | 23 | 2% Pluronic |
| Exxon 1605 PP | Lens C | 30 | 16 | 2% Pluronic |
| Exxon 1605 PP | Lens C | 77 | 0 | PVP K90 (5%) + Epolene E43 (5%) |
| Exxon 1605 PP | Lens B | 50 | 0 | PVP K90 (5%) + Epolene E43 (5%) |
| Exxon 1605 PP | Lens C | 62 | 0 | PVP K90 (5%) + Epolene E43 (1.5%) |
| Exxon 1605 PP | Lens B | 50 | 0 | PVP K90 (5%) + Epolene E43 (1.5%) |
| Exxon 1605 PP | Lens C | 65 | 0 | PVP K90 (2.5%) + Epolene E43 (1.25%) |
| Exxon 1605 PP | Lens B | 50 | 0 | PVP K90 (2.5%) + Epolene E43 (1.25%) |
| Exxon 1605 PP | Lens C | 115 | 10 | PVP K90 (1%) + Epolene E43 (1%) |
| Exxon 1605 PP | Lens B | 100 | 11 | PVP K90 (1%) + Epolene E43 (1%) |
| Exxon 1605 PP | Lens C | 30 | 0 | PVP K29/31 (5%) |
| Exxon 1605 PP | Lens C | 30 | 0 | PVP K60 (5%) |
| Exxon 1605 PP | Lens B | 50 | 0 | PVP K90 (1%) + Rough Bowl (UP) |
| Exxon 1605 PP | Lens C | 50 | 0 | PVP K90 (1%) + Rough Bowl (UP) |
| Exxon 1605 PP | Lens B | 170 | 0 | Epolene E43 (1%) + Rough Bowl |
| Exxon 1605 PP | Lens C | 200 | 0 | Epolene E43 (1%) + Rough Bowl |

## Claims

1. A package for storing contact lenses in a solution, comprising a molded base wherein the molded base comprises an additive, wherein the additive is polyvinylpyrrolidinone (PVP), wherein the additive is present at a concentration of 1 weight percent to 5 weight percent, wherein the package contains a contact lens, wherein the contact lens is in a solution and wherein the molded base comprises polypropylene mixed with said additive, provided that the contact lens is not a contact lens consisting of acquafilcon A coated with polyHema.

2. A method of reducing the adherence of a contact lens to its packaging, comprising storing said contact lens in a solution in a package comprising a molded base wherein said molded base comprises an additive, wherein said additive is polyvinylpyrrolidinone (PVP) and wherein the molded base comprises polypropylene mixed with said additive, wherein the additive is present at a concentration of 1 weight percent to 5 weight percent, provided that the contact lens is not a contact lens consisting of acquafilcon A coated with po lyHema.

3. The package of claim 1 or the method of claim 2 wherein the additive is polyvinylpyrrolidinone (PVP) KD90.

4. The package of claim 3 wherein the polyvinylpyrrolidinone (PVP) concentration is 1.0%.

5. The package of claim 1 or the method of claim 2 wherein the contact lens is a contact lens which comprises balafilcon A, lotrafilcon A, galyfilcon A, or senofilcon A.

6. The package of claim 5 wherein the contact lens comprises 3-methacryloxy-2-hydroxypropyloxy propylbis (trimethylsiloxy)methylsilane (Simma 2) and monomethacryloxypropyl terminated polydimethylsiloxane having a molecular weight of 800-1000 (mPDMS).

7. The package of claim 5 or the method of claim 2 wherein the contact lens comprises 3-methacryloxy-2-hydroxypropyloxy propylbis (trimethylsiloxy)methylsilane (Simma 2).

8. The package of claim 1 or the method of claim 2 further comprising a cavity formed in said molded base wherein said cavity comprises an inner surface, wherein said inner surface has an average roughness (Rₐ) of 0.5 µm to 20 µm.

9. The package of claim 8 wherein the inner surface has an average roughness (Rₐ) of 1.8 µm to 4.5 µm.

10. The package of claim 8 wherein the inner surface has an average roughness (Rₐ) of 1.9 µm to 2.1 µm.

11. The package of claim 8 wherein the inner surface has an average roughness (Rₐ) of 0.5 µm to 0.8 µm.

12. The package or method of claim 8 wherein the average roughness (Rₐ) of the inner surface is 0.5 µm to 0.8 µm and the concentration of polyvinylpyrrolidinone (PVP) is 1%.

13. The package or method of claim 8 wherein the inner surface has an average roughness (Rₐ) of 1.9 µm to 2.1 µm and the concentration of polyvinylpyrrolidinone (PVP) is 1%.

## Patentansprüche

1. Verpackung zum Aufbewahren von Kontaktlinsen in einer Lösung, umfassend eine geformte Basis, wobei die geformte Basis einen Zusatzstoff umfasst, wobei der Zusatzstoff Polyvinylpyrrolidinon (PVP) ist, wobei der Zusatzstoff in einer Konzentration von 1 Gewichtsprozent bis 5 Gewichtsprozent vorhanden ist, wobei die Verpackung eine Kontaktlinse enthält, wobei sich die Kontaktlinse in einer Lösung befindet und wobei die geformte Basis Polypropylen gemischt mit dem Zusatzstoff umfasst, mit der Maßgabe, dass die Kontaktlinse keine Kontaktlinse ist, die aus Acquafilcon A überzogen mit polyHema besteht.

2. Verfahren zum Verringern des Anhaftens einer Kontaktlinse an ihre Verpackung, umfassend Aufbewahren der Kontaktlinse in einer Lösung in einer Verpackung umfassend eine geformte Basis, wobei die geformte Basis einen Zusatzstoff umfasst, wobei der Zusatzstoff Polyvinylpyrrolidinon (PVP) ist und wobei die geformte Basis Polypropylen gemischt mit dem Zusatzstoff umfasst, wobei der Zusatzstoff in einer Konzentration von 1 Gewichtsprozent bis 5 Gewichtsprozent vorhanden ist, mit der Maßgabe, dass die Kontaktlinse keine Kontaktlinse ist, die aus Acquafilcon A überzogen mit polyHema besteht.

3. Verpackung gemäß Anspruch 1 oder Verfahren gemäß Anspruch 2, wobei der Zusatzstoff Polyvinylpyrrolidinon (PVP) KD90 ist.

4. Verpackung gemäß Anspruch 3, wobei die Polyvinylpyrrolidinon(PVP)-Konzentration 1,0 % beträgt.

5. Verpackung gemäß Anspruch 1 oder Verfahren gemäß Anspruch 2, wobei die Kontaktlinse eine Kontaktlinse ist, die Balafilcon A, Lotrafilcon A, Galyfilcon A oder Senofilcon A umfasst.

6. Verpackung gemäß Anspruch 5, wobei die Kontaktlinse 3-Methacryloxy-2-hydroxypropyloxypropylbis(trimethylsiloxy)-methylsilan (Simma 2) und Monomethacryloxypropylterminiertes Polydimethylsiloxan mit einem Molekulargewicht von 800-1000 (mPDMS) umfasst

7. Verpackung gemäß Anspruch 5 oder Verfahren gemäß Anspruch 2, wobei die Kontaktlinse 3-Methacryloxy-2-hydroxypropyloxypropylbis(trimethylsiloxy)-methylsilan (Simma 2) umfasst.

8. Verpackung gemäß Anspruch 1 oder Verfahren gemäß Anspruch 2, ferner umfassend eine in der geformten Basis gebildete Kammer, wobei die Kammer eine Innenoberfläche umfasst, wobei die Innenoberfläche eine mittlere Rauigkeit (Rₐ) von 0,5 µm bis 20 µm aufweist.

9. Verpackung gemäß Anspruch 8, wobei die Innenoberfläche eine mittlere Rauigkeit (Rₐ) von 1,8 µm bis 4,5 µm aufweist.

10. Verpackung gemäß Anspruch 8, wobei die Innenoberfläche eine mittlere Rauigkeit (Rₐ) von 1,9 µm bis 2,1 µm aufweist.

11. Verpackung gemäß Anspruch 8, wobei die Innenoberfläche eine mittlere Rauigkeit (Rₐ) von 0,5 µm bis 0,8 µm aufweist.

12. Verpackung oder Verfahren gemäß Anspruch 8, wobei die mittlere Rauigkeit (Rₐ) der Innenoberfläche 0,5 µm bis 0,8 µm beträgt und die Konzentration von Polyvinylpyrrolidinon (PVP) 1 % beträgt.

13. Verpackung oder Verfahren gemäß Anspruch 8, wobei die Innenoberfläche eine mittlere Rauigkeit (Rₐ) von 1,9 µm bis 2,1 µm aufweist und die Konzentration von Polyvinylpyrrolidinon (PVP) 1 % beträgt.

## Revendications

1. Emballage pour la conservation de lentilles de contact dans une solution, comprenant une base moulée, la base moulée comprenant un additif, l'additif étant la polyvinylpyrrolidone (PVP), l'additif étant présent à une concentration de 1 % en poids à 5 % en poids, l'emballage contenant une lentille de contact, la lentille de contact se trouvant dans une solution, et la base moulée comprenant du polypropylène mélangé avec ledit additif, sous réserve que la lentille de contact ne soit pas une lentille de contact constituée d'aquafilcon A revêtu de polyHema.

2. Procédé de réduction de l'adhérence d'une lentille de contact à son emballage, comprenant la conservation de ladite lentille de contact dans une solution dans un emballage comprenant une base moulée, ladite base moulée comprenant un additif, ledit additif étant la polyvinylpyrrolidone (PVP), et la base moulée comprenant du polypropylène mélangé avec ledit additif, l'additif étant présent à une concentration de 1 % en poids à 5 % en poids, sous réserve que la lentille de contact ne soit pas une lentille de contact constituée d'aquafilcon A revêtu de polyHema.

3. Emballage selon la revendication 1 ou procédé selon la revendication 2, dans lequel l'additif est la polyvinylpyrrolidone (PVP) KD90.

4. Emballage selon la revendication 3, dans lequel la concentration de polyvinylpyrrolidone (PVP) est de 1,0 %.

5. Emballage selon la revendication 1 ou procédé selon la revendication 2, dans lequel la lentille de contact est une lentille de contact qui comprend du balafilcon A, du lotrafilcon A, du galyfilcon A, ou du senofilcon A.

6. Emballage selon la revendication 5, dans lequel la lentille de contact comprend du 3-méthacryloxy-2-hydroxypropyloxypropyl-bis(triméthylsiloxy)méthylsilane (Simma 2) et un polydiméthylsiloxane à terminaison monométhacryloxypropyle ayant une masse moléculaire de 800 à 1 000 (mPDMS).

7. Emballage selon la revendication 5 ou procédé selon la revendication 2, dans lequel la lentille de contact comprend du 3-méthacryloxy-2-hydroxypropyloxypropyl-bis(triméthylsiloxy)méthylsilane (Simma 2).

8. Emballage selon la revendication 1 ou procédé selon la revendication 2, comprenant en outre une cavité formée dans ladite base moulée, ladite cavité comprenant une surface interne, ladite surface interne ayant une rugosité moyenne (Rₐ) de 0,5 µm à 20 µm.

9. Emballage selon la revendication 8, dans lequel la surface interne a une rugosité moyenne (Rₐ) de 1,8 µm à 4,5 µm.

10. Emballage selon la revendication 8, dans lequel la surface interne a une rugosité moyenne (Rₐ) de 1,9 µm à 2,1 µm.

11. Emballage selon la revendication 8, dans lequel la surface interne a une rugosité moyenne (Rₐ) de 0,5 µm à 0,8 µm.

12. Emballage ou procédé selon la revendication 8, dans lequel la rugosité moyenne (Rₐ) de la surface interne est de 0,5 µm à 0,8 µm et la concentration de polyvinylpyrrolidone (PVP) est de 1 %.

13. Emballage ou procédé selon la revendication 8, dans lequel la surface interne a une rugosité moyenne (Rₐ) de 1,9 µm à 2,1 µm et la concentration de polyvinylpyrrolidone (PVP) est de 1 %.
